# EUROPÄISCHE PATENTSCHRIFT

(11) **EP 0 365 973 B1**
(45) Veröffentlichungstag und Bekanntmachung des Hinweises auf die Patenterteilung: **15.12.1993**
(21) Anmeldenummer: 89119222.1
(22) Anmeldetag: 17.10.1989
(51) Int. Cl.: C07C 205/57, C07C 69/95, C07C 50/36, C07H 15/252, A61K 31/70

(54) **Verfahren zur Herstellung von 4-0-Alkyl-Rhodomycinen**
Process for preparing 4-0-alkyl-rhodomycins
Procédé pour la préparation de rhodomycines 0-alkylées en position 4

(30) Priorität: 22.10.1988 DE 3836122
(43) Veröffentlichungstag der Anmeldung: 02.05.1990
(73) Patentinhaber: BEHRINGWERKE Aktiengesellschaft, 35001 Marburg (DE)
(72) Erfinder: Kolar, Cenek, Dr., D-3550 Marburg (DE)

(56) Entgegenhaltungen:
- EP-A- 0 277 310
- EP-A- 0 286 926
- DE-A- 3 641 833
- CHEMICAL ABSTRACTS, Band 105, no. 1, July 7, 1986, Columbus, Ohio, USA YOSHIMOTO, AKIHIRO et al.: "Manufacture of novel anthra- cyline antibiotics with Streptomyces D788 mutant." Seite 502, Zusammen- fassung-Nr. 5172d
- F. ARCAMONE, "Doxorubicin, Anticancer Antibiotics", Academic Press, 1981, pages 289-299

## Beschreibung

Die vorliegende Erfindung bezieht sich auf ein neues Verfahren zur Herstellung von 4-0-Alkyl-rhodomycinen, besonders 4-0-Alkyl-β und epsilon-rhodomycinonen und deren Verwendung zur Herstellung von 7-0-Glycosyl-rhodomycinen, die aufgrund ihrer zytostatischen Wirksamkeit für die Behandlung von Krebserkrankungen geeignet sind.

Anthracycline sind in der Fachliteratur beschrieben. Von der Doxorubicin-Daunorubicin-Gruppe der Anthracycline ist bekannt, daß die 4-0-Methyl-Substitution in den Anthracyclinen für die antitumorale Wirkung und Tumorselektivität erforderlich ist. Die entsprechenden 4-Hydroxy-Derivate wie Carminomycine sowie β-Rhodomycine sind zytotoxischer und weniger Tumor-selektiv.

Der Erfindung liegt die Aufgabe zugrunde, ein Verfahren zu entwickeln, das 4-0-Alkyl-rhodomycinon-Derivate in guten Ausbeuten liefert und eine vereinfachung gegenüber dem bekannten Verfahren (Doxorubicin, Anti-Cancer Antibiotics, Herausgeber: Frederico Arcamone, Academic Press, New York and London, 1981, Seiten 289-299) bedeutet, sowie die Ausarbeitung einer Schutzgruppenchemie an 4-0-Alkyl-rhodomycinonen, die es erlaubt, das Aglycon zur Herstellung der 7-0-Glycosyl-rhodomycinone zu verwenden. Das vorliegende Verfahren zur Herstellung der 4-0-Alkyl-rhodomycine basiert auf der überaschenden Erkenntnis, daß sich ein ungeschütztes Rhodomycinon-Aglycon mit 1,3-Dichlor-1,1,3,3-tetraisopropyldisiloxan zu einem 6,7-O-(Tetraisopropyldisiloxan-1,3-diyl)-rhodomycinon und anschließend mit einem Trialkylsilylhalogenid zu einer 10-O-Trialkylsilyl-Verbindung umsetzen läßt. Hierdurch kann die folgende Alkylierung nur an den phenolischen Hydroxygruppen an C-4- und C-11-Atomen ablaufen, wobei überraschenderweise die Alkylierung der phenolischen Gruppe am C-4-Atom bevorzugt verläuft.

Diese Aufgabe wird erfindungsgemäß gelöst durch das Verfahren zur Herstellung eines Rhodomycin-Derivates der Formel I 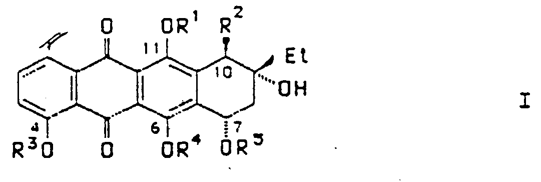
worin
R¹ H, C₁-C₄-Alkyl oder eine Acylschutzgruppe,
R² OH, COOCH₃, O-Si(C₁-C₄-Alkyl)₃ oder eine O-Acylschutzgruppe,
R³ C₁-C₄-Alkyl,
R⁴ und R⁵ H oder
R⁴ und R⁵ zusammen eine Tetraisopropyldisiloxan-1,3-diyl-Schutzgruppe und
Acylschutzgruppe Acetyl, Mono-, Di-, oder Trihalogenacetyl mit Fluor oder
Chlor als Halogen, Benzoyl oder p-Nitrobenzoyl sind,
**dadurch gekennzeichnet,** daß man ein Rhodomycinon-Derivat der Formel I,
worin
R¹ H,
R² OH oder COOCH₃,
R³, R⁴ und R⁵ H sind
mit 1,3-Dichlor-1,1,3,3-tetra-isopropyldisiloxan in Gegenwart einer Base wie Pyridin oder Dimethylaminopyridin und eines organischen Lösungsmittels wie Dichlormethan oder Chloroform bei einer Temperatur zwischen 15° C und 80° C zu einer 6,7-Tetraisopropyldisiloxan-1,3-diyl)-rhodomycinon-Verbindung der Formel I umsetzt und wobei anschließend die Verbindung, die am C-10 Atom den Rest R² = OH enthält unter gleichen Reaktionsbedingungen mit Tri-(C₁-C₄)-alkylsilylhalogenid umsetzt, dann die sililierte Zwischenverbindung der Formel I,
worin
R¹ und R³ H,
R² COOCH₃ oder O-Si(C₁-C₄-alkyl)₃ und
R⁴ und R⁵ zusammen eine Tetraisopropyldisiloxan-1,3-diyl-Gruppe sind,
mit C₁-C₄-Alkylbromid oder -jodid in Gegenwart eines Alkalicarbonats und eines organischen Lösungsmittels wie Aceton oder Dioxan zu einer Mono- oder Dialkylverbindung der Formel I,
worin
R¹ C₁-C₄-alkyl,
R³ H oder C₁-C₄-alkyl sind und
R², R⁴ und R⁵ unverändert bleiben,
alkyliert, dann bei einer Verbindung, die eine O-Trialkylsilylgruppe am C-10 Atom enthält, diese Gruppe selektiv säure-hydrolytisch, bevorzugt mit Salzsäure abspaltet und die entstandene Verbindung mit dem Rest R² gleich Hydroxygruppe mit einem Acylierungmittel ausgewählt aus der Gruppe Acetylchlorid, Mono-, Di-, oder Trihalogenacetylchlorid oder -anhydrid mit Fluor oder Chlor als Halogen, Benzoylchlorid oder p-Nitrobenzoylchlorid in Gegewart einer Base wie Pyridin, Triethylamin oder Dimethylaminopyridin und eines Lösungsmittels wie Dichlormethan oder Chloroform zu einer Mono- oder Diacylverbindung acyliert und anschließend bei einer Tetraisopropyldisiloxan-Verbindung der Formel I,
worin
R¹ H, C₁-C₄-Alkyl oder eine Acylschutzgruppe,
R² COOCH₃ oder eine O-Acylschutzgruppe,
R³ C₁-C₄-Alkyl und
R⁴ und R⁵ zusammen eine Tetraisopropyldisiloxan-1,3-diyl-Schutzgruppe sind, die Tetraisopropyldisiloxan-1,3-diyl-Schutzgruppe mittels eines Ammoniumfluorides, bevorzugt Tetrabutylammoniumfluorid, in einem polarem Lösungsmittel wie Tetrahydrofuran oder Dioxan abspaltet, wobei eine Verbindung der Formel I,
worin
R¹ H, C₁-C₄-Alkyl oder eine Acylschutzgruppe,
R² COOCH₃ oder eine O-Acylschutzgruppe,
R³ C₁-C₄-Alkyl,
R⁴ und R⁵ H und
Acylschutzgruppe Acetyl, Mono-, Di-, oder Trihalogenacetyl mit Fluor oder
Chlor als Halogen, Benzoyl oder p-Nitrobenzoyl sind,
entsteht.

Bevorzugt werden Verbindungen der Formel I hergestellt,
worin
R¹ H, C₁-C₄-Alkyl oder eine Acylschutzgruppe,
R² COOCH₃, O-Si(C₁-C₄-Alkyl)₃ oder eine O-Acylschutzgruppe,
R³ C₁-C₄-Alkyl und
R⁴ und R⁵ zusammen eine Tetraisopropyldisiloxan-1,3-diyl-Schutzgruppe und
Acylschutzgruppe Acetyl, Mono-, Di-, oder Trihalogenacetyl mit Fluor oder
Chlor als Halogen, Benzoyl oder p-Nitrobenzoyl sind,
Besonders bevorzugt werden Verbindungen der Formel I hergestellt,
worin
R¹ H, C₁-C₄-Alkyl oder eine Acylschutzgruppe,
R² OH, COOCH₃, O-Si(C₁-C₄-Alkyl)₃ oder eine O-Acylschutzgruppe,
R³ C₁-C₄-Alkyl und
R⁴ und R⁵ H sind,
In Schema 1 ist das der Erfindung zugrunde liegende Verfahren dargestellt.

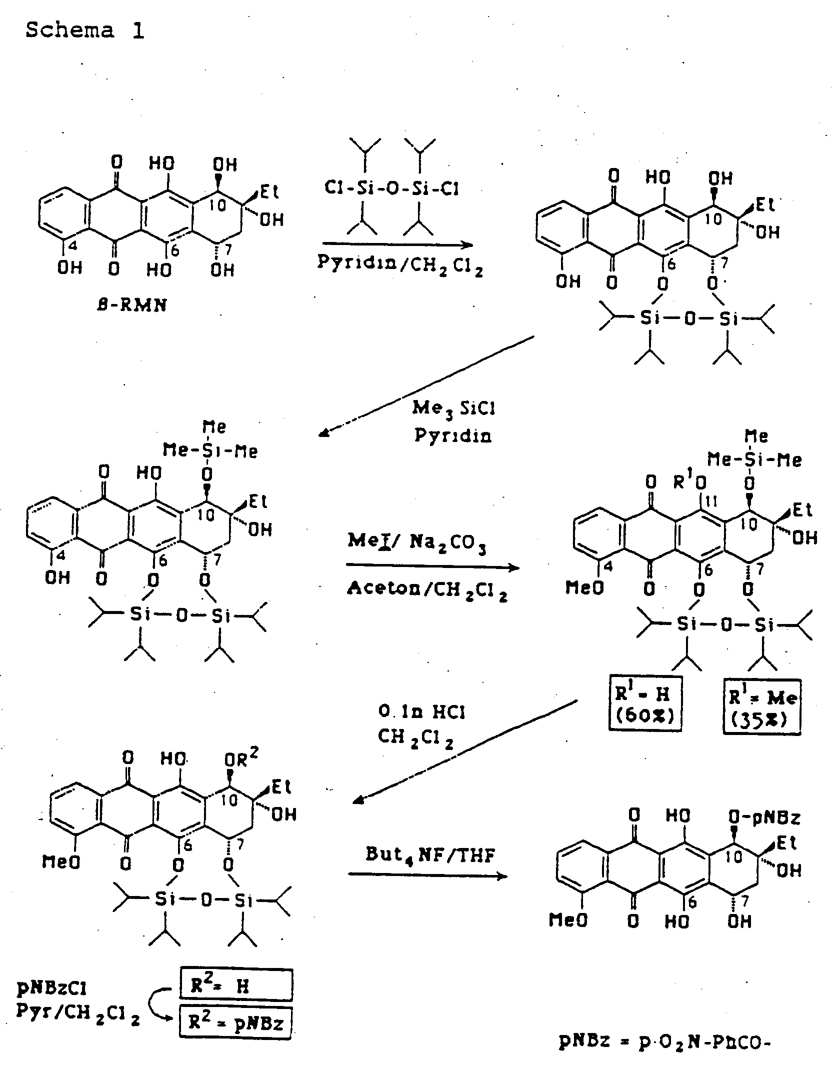

### Beispiele

In den folgenden Beispielen wird die vorliegende Erfindung näher beschrieben, ohne sie hierbei einzuschränken.

Die Struktur der in den folgenden Beispielen beschriebenen Verbindungen wurden mittels NMR- und MS-Analytik ermittelt. Der Verlauf der Reaktionen sowie die chemische Reinheit der Verbindungen wurde dünnschichtchromatographisch oder HPLC-mäßig untersucht.

### Beispiel 1

### Einführung von Silylschutzgruppen in ein Rhodomycinon in die Position 6,7 gegebenenfalls 10

### 6,7-O-(1,1,3,3-Tetraisopropyldisiloxan-1,3-diyl)-β-rhodomycinon (Verbindung 1)

10 g (25.8 mmol) β-Rhodomycinon wurden in 300 ml Pyridin/Dichlormethan (1:1) gelöst und mit 12.2 ml (1.5 äq) 1,3-Dichlor-1,1,3,3-tetraisopropyldisiloxan, gelöst in 150 ml Dichlormethan bei 0° C unter Feuchtigkeitsausschluß versetzt. Der Reaktionsansatz wurde 24 Stunden bei Raumtemperatur dann weitere 72 Stunden bei 60° C gerührt. Nach der weiteren Zugabe von 6.5 ml (0.8 äq) 1,3-Dichlor-1,1,3,3-tetraisopropyldisiloxan wurde der Reaktionsansatz weitere 72 Stunden bei 60° C gerührt. Die Reaktionsmischung wurde mit 50 ml Methanol versetzt, in Vakuum eingedampft und mit Toluol zweimal nachdestilliert. Der verbleibende Rückstand wurde säulenchromatographisch (Kieselgel; Elutionsmittel: Chloroform/Essigsäureethylester 20:1) gereinigt.
Ausbeute: 10.8 g (67 %); Schmelzpunkt: 115-117° C
(alpha)_{D} = +425° (c = 0.2 in Chloroform)
¹H-NMR (90 MHz, CDCl₃, delta) : 13.91 und 13.07 (s, PhOH), 7.82 (br d, H-1), 7.65 (t, H-2), 7.30 (br d, H-3), 5.28 (br s, H-7), 4.96 (s, H-10), 4.75 (s, 9-OH), 3.66-3.54 (m, Alkyl-Si)

### 6,7-O-(1,1,3,3-Tetraisopropyldisiloxan-1,3-diyl)-epsilon-rhodo-mycinon (Verbindung 2)

5 g (11.67 mmol) epsilon-Rhodomycinon und 9.2 g (2.5 äq) 1,3-Dichlor-1,1,3,3-tetraisopropyldisiloxan wurden in 150 ml Dichlormethan/Pyridin (1:1) gelöst. Nach 8 Tagen Reaktionszeit wurde die Titelverbindung, wie in der Vorschrift Zur Herstellung der Verbindung 1 beschrieben, isoliert.
Ausbeute: 6.1 g (78 %)

### 6,7-O-(1,1,3,3-Tetraisopropyldisiloxan-1,3-diyl)-10-O-trimethylsilyl-β-rhodomycinon (Verbindung 3)

19.0 g (30.2 mmol) Verbindung 1 wurden in 250 ml Dichlormethan und 250 ml Pyridin gelöst und mit 4.2 g (1.5 äq) Chlortrimethylsilan, gelöst in 50 ml Dichlormethan, bei 0° C versetzt. Die Reaktionsmischung wurde 1 Stunde bei Raumtemperatur gerührt. Dann wurde die Reaktionsmischung in Vakuum eingedampft und zweimal mit Toluol nachdestilliert. Der Rückstand wurde säulenchromatographisch (Kieselgel; Elutionsmittel: Dichlormethan/Petrolether 7:3) gereinigt.
Ausbeute: 16.3 g (77 %); Schmelzpunkt: 194-196° C
(alpha)_{D} = +530° (c = 0.2 in Chloroform)
¹H-NMR (300 MHz, CDCl₃, delta) : 13.87 und 13.12 (s, PhOH), 7.79 (dd, H-1), 7.61 (t, H-2), 7.25 (dd, H-3), 5.28 (dd, H-7), 2.13 (dd, H-8a), 2.06 (dd, HR-8b), 4.86 (br s, H-10), 1.64 (m, H-13a und H-13b), 1.02 (t, H-14), 0.2 und 1.2-1.3 (m, Alkyl-Si)

### Beispiel 2

Alkylierung von einem Rhodomycinon in der Position 4 gegebenenfalls 11.

### 4-0-Methyl-6,7-O-(1,1,3,3-tetraisopropyldisiloxan-1,3-diyl)-10-O-trimethylsilyl-β-rhodomycinon (Verbindung 4) und

### 4,11-Di-O-methyl-6,7-O-(1,1,3,3-tetraisopropyldisiloxan-1,3-diyl)-10-O-trimethylsilyl-β-rhodomycinon (Verbindung 5)

### Verfahren a):

0.75 g (1.07 mmol) Verbindung 3 wurden in 30 ml Aceton und 7 ml Dichlormethan gelöst und mit 4.4 g (30 äq) Kaliumcarbonat und 10 ml (150 äq) Methyljodid versetzt. Die Reaktionsmischung wurde 3 Tage bei Raumtemperatur gerührt, wobei der Reaktionsverlauf dünnschichtchromatographisch verfolgt wurde. Der Reaktionsansatz wurde mit Dichlormethan versetzt und nacheinander mit Wasser, 1 n Salzsäure und Wasser ausgewaschen. Die organische Phase wurde über Natriumsulfat getrockent und in Vakuum eingedampft. Die in dem Rückstand vorgandenen Verbindungen 6 und 7 wurden säulenchromatographisch an 35 g Kieselgel mit Dichlormethan als Eluenz getrennt.

### Verbindung 4:

Ausbeute: 0.46 g (60 %); Schmelzpunkt: 213-214° C
(alpha)_{D} = +472° (c = 0.05 in Chloroform)
¹H-NMR (300 MHz, CDCl₃_{'} delta): 13.22 (s, PhOH), 7.89 (dd, H-1), 7.65 (t, H-2), 7.29 (dd, H-3), 5.35 (dd, H-7), 2.17 (dd, H-8a), 2.07 (dt, H-8b), 4.89 (d, H-10), 1.39 (m, H-13a), 1.15 (m, H-13b), 1.05 (t, H-14), 3.96 (s, OMe), 0.1 und 1.18-1.33 (m, Alkyl-Si), 4.45 (s, 9-OH)

### Verbindung 5:

Ausbeute: 0.27 g (35 %); Schmelzpunkt: 103-105° C
(alpha)_{D} = +124° (c = 0.05 in Chloroform)
¹H-NMR (400 MHz, CDCl₃, delta): 7.72 (dd, H-1), 7.59 (t, H-2), 7.21 (dd, H-3), 5.34 (dd, H-7), 2.17 (dd, H-8a), 2.05 (dt, H-8b), 4.70 (d, H-10), 1.40 (m, H-13a), 1.31 (m, H-13b), 1.04 (t, H-14), 3.93 und 3.91 (s, OMe), 0.1 und 0.9-1.3 (Alkyl-Si), 4.50 (s, 9-OH)

### Verfahren b):

1 g (1.42 mmol) Verbindung 3 wurden in 4.5 ml Aceton und 18 ml Dichlormethan gelöst und mit 4.2 g (12.9 mmol) Cesiumcarbonat und 5 ml (71.5 mmol) Methyljodid unter Rühren bei 4° C versetzt. Nach 3 Tagen Reaktionszeit wurde der ReaktionsansatZ wie oben beschrieben aufgearbeitet, wobei die Verbindung 4 (Ausbeute: 0.76 g (75 %)) und die Verbindung 5 (Ausbeute: 0.1 g (10 %)) isoliert wurden.

### 4-O-Methyl-6,7-O-(1,1,3,3-tetraisopropyl-disiloxan-1,3-diyl)-epsilon-rhodomycinon (Verbindung 6)

Ausgehend von 6.03 g (9 mmol) Verbindung 2, 37.49 g (30 äq) Kaliumcarbonat und 56.5 ml (900 mmol) Methyljodid wurde die Titelverbindung nach der Vorschrift zur Herstellung der Verbindung 4 hergestellt.
Ausbeute: 3.2 g (52 %)

### Beispiel 3

### Selektive Entblockierung der Trialkylsilyl-Schutzgruppe

### 4-O-Methyl-6,7-O-(1,1,3,3-tetraisopropyldisiloxan-1,3-diyl)-β-rhodomycinon (Verbindung 7)

2 g (2.8 mmol) Verbindung 4 wurden in 100 ml Dichlormethan und 100 ml Methanol gelöst und mit 20 ml 0.1 n Salzsäure versetzt. Nach 30 min Rühren bei Raumtemperatur wurde der Reaktionsansatz mit Dichlormethan verdünnt und mit Wasser ausgeschüttelt. Die organische Phase wurde mit Natriumsulfat getrocknet und in Vakuum eingedampft. Der Rückstand wurde noch über 50 g Kieselgel (Eluenz: Dichlormethan/ Aceton 20:1) filtriert.
Ausbeute: 1.7 g (96 %); Schmelzpunkt: 166-168° C
(alpha)_{D} = +386° (c = 0.05 in Chloroform)

### Beispiel 4

### Einführung von Acylschutzgruppen

### 4-O-Methyl-10-O-p-nitrobenzoyl-6,7-O-(1,1,3,3-tetraisopropyldisiloxan-1,3-diyl)-β-rhodomycinon (Verbindung 8)

0.68 g (1.05 mmol) Verbindung 7 wurden in 40 ml Chloroform/ Pyridin (2:1) gelöst und mit 0.3 g (1.5 äq) p-Nitrobenzoylchlorid versetzt. Nach 15 Stunden Rühren bei 0° C wurden 10 ml Methanol der Reaktionsmischung zugegeben. Der Reaktionsansatz wurde in Vakuum eingedampft und mit Toluol nachdestilliert. Der in Chloroform gelöste Rückstand wurde zweimal mit Wasser gewaschen, die organische Phase wurde über Natriumsulfat getrocknet und in Vakuum eingedampft. Das verbleibende Rohprodukt wurde noch säulenchromatographisch über 120 g Kieselgel (Eluenz: Petrolether/Dichlormethan/Aceton 17:10:1) gereinigt.
Ausbeute: 340 mg (81.1 %); Schmelzpunkt: 114-116° C
(alpha)_{D} = +384° (c = 0.05 in Chloroform)
¹H-NMR (300 MHz, CDCl₃, delta): 13.02 (s, PhOH), 7.84 (dd, H-1), 7.65 (t, H-2, 7.32 (dd, H-3), 5.51 (dd, H-7), 2.38 (dt, H-8a), 2.07 (dd, H-8b), 6.56 (d, H-10), 1.50 (m, H-13a), 1.83 (m, H-13b), 1.07 (t, H-14), 3.98 (s, 4-OMe), 8.08 und 8.22 (m, Aryl), 0.64-1.2 (m, Alkyl-Si)

### 10,11-O-Bis-(p-nitrobenzoyl)-4-O-methyl-6,7-O-(1,1,3,3-tetraisopropyldisiloxan-1,3-diyl)-β-rhodomycinon (Verbindung 9)

0.34 g (0.529 mmol) Verbindung 7 wurden mit 0.3 g (3 äq) p-Nitrobenzoylchlorid, wie oben beschrieben, zu der Titelverbindung umgesetzt.
Ausbeute: 0.35 g (72 %)

### Beispiel 5

### Entblockierung von Tetraisopropyldisiloxan-1,3-diyl-Schutzgruppe

### 4-O-Methyl-10-O-p-nitrobenzoyl-β-rhodomycinon (Verbindung 10)

0.6 g (0.76 mmol) Verbindung 8 wurden in 30 ml THF gelöst und bei 0° C mit 1 ml einer 1 molaren Tetrabutylammoniumfluorid THF-Lösung versetzt. Nach 30 min Rühren wurde der Reaktionsansatz mit 30 ml 0.1 n Salzsäure versetzt und zweimal mit Chloroform extrahiert. Die organische Phase wurde über Natriumsulfat getrocknet und in Vakuum eingedampft. Das erhaltene Rohprodukt wurde über 65 g Kieselgel (Laufmittel: Dichlormethan/Aceton 15:1) chromatographisch gereinigt.
Ausbeute: 0.378 g (91 %); Schmelzpunkt: 153-155° C
(alpha)_{D} = +275° (c = 0.02 in Chloroform)

### 10,11-O-Bis-(p-nitrobenzoyl)-4-O-methyl-β-rhodomycinon (Verbindung 11)

0.15 g (0.159 mmol) Verbindung 9 wurden mit 0.2 ml einer 1 molaren Tetrabutylammoniumfluorid THF-Lösung, wie oben beschrieben, zu der Titelverbindung umgesetzt.
Ausbeute: 95.8 mg (88 %)

### 4-O-Methyl-epsilon-rhodomycinon (Verbindung 12)

Ausgehend von 3.2 g (4.6 mmol) Verbindung 6 wurde die Titelverbindung nach der Vorschrift zur Herstellung der Verbindung 10 hergestellt.
Ausbeute: 1.6 g (83 %); FAB m/e = 443 (M+H⁺)

## Patentansprüche

1. Verfahren zur Herstellung eines Rhodomycin-Derivates der Formel I 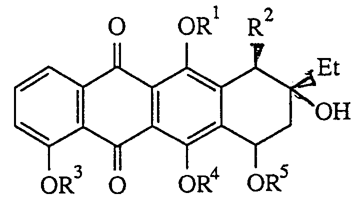 worin
R¹ H, C₁-C₄-Alkyl oder eine Acylschutzgruppe,
R² OH, COOCH₃, O-Si(C₁-C₄-Alkyl)₃ oder eine O-Acylschutzgruppe,
R³ C₁-C₄-Alkyl,
R⁴ und R⁵ H oder
R⁴ und R⁵ zusammen eine Tetraisopropyldisiloxan-1,3-diyl-Schutzgruppe und Acylschutzgruppe Acetyl, Mono-, Di-, oder Trihalogenacetyl mit Fluor oder Chlor als Halogen, Benzoyl oder p-Nitrobenzoyl sind,
**dadurch gekennzeichnet**, daß man ein Rhodomycinon-Derivat der Formel I,
worin
R¹ H,
R² OH oder COOCH₃,
R³, R⁴ und R⁵ H sind
mit 1,3-Dichlor-1,1,3,3-tetra-isopropyldisiloxan in Gegenwart einer Base wie Pyridin oder Dimethylaminopyridin und eines organischen Lösungsmittels wie Dichlormethan oder Chloroform bei einer Temperatur zwischen 15° C und 80° C zu einer 6,7-Tetraisopropyldisiloxan-1,3-diyl)-rhodomycinon-Verbindung der Formel I umsetzt und wobei anschließend die Verbindung, die am C-10 Atom den Rest R² = OH enthält unter gleichen Reaktionsbedingungen mit Tri-(C₁-C₄)-alkylsilylhalogenid umsetzt, dann die sililierte Zwischenverbindung der Formel I,
worin
R¹ und R³ H,
R² COOCH₃ oder O-Si(C₁-C₄-alkyl)₃ und
R⁴ und R⁵ zusammen eine Tetraisopropyldisiloxan-1,3-diyl-Gruppe sind,
mit C₁-C₄-Alkylbromid oder -jodid in Gegenwart eines Alkalicarbonats und eines organischen Lösungsmittels wie Aceton oder Dioxan zu einer Mono- oder Dialkylverbindung der Formel I,
worin
R¹ C₁-C₄-alkyl,
R³ H oder C₁-C₄-alkyl sind und
R², R⁴ und R⁵ unverändert bleiben,
alkyliert, dann bei einer Verbindung, die eine O-Trialkylsilylgruppe am C-10 Atom enthält, diese Gruppe selektiv säure-hydrolytisch, bevorzugt mit Salzsaure aospaltet und die entstandene Verbindung mit dem Rest R² gleich Hydroxygruppe mit einem Acylierungmittel ausgewählt aus der Gruppe Acetylchlorid, Mono-, Di-, oder Trihalogenacetylchlorid oder -anhydrid mit Fluor oder Chlor als Halogen, Benzoylchlorid oder p-Nitrobenzoylchlorid in Gegewart einer Base wie Pyridin, Triethylamin oder Dimethylaminopyridin und eines Lösungsmittels wie Dichlormethan oder Chloroform zu einer Mono- oder Diacylverbindung acyliert und anschließend bei einer Tetraisopropyldisiloxan-Verbindung der Formel I,
worin
R¹ H, C₁-C₄-Alkyl oder eine Acylschutzgruppe,
R² COOCH₃ oder eine O-Acylschutzgruppe,
R³ C₁-C₄-Alkyl und
R⁴ und R⁵ zusammen eine Tetraisopropyldisiloxan-1,3-diyl-Schutzgruppe sind, die Tetraisopropyldisiloxan-1,3-diyl-Schutzgruppe mittels eines Ammoniumfluorides, bevorzugt Tetrabutylammoniumfluorid, in einem polarem Lösungsmittel wie Tetrahydrofuran oder Dioxan abspaltet, wobei eine Verbindung der Formel I,
worin
R¹ H, C₁-C₄-Alkyl oder eine Acylschutzgruppe,
R² COOCH₃ oder eine O-Acylschutzgruppe,
R³ C₁-C₄-Alkyl,
R⁴ und R⁵ H und
Acylschutzgruppe Acetyl, Mono-, Di-, oder Trihalogenacetyl mit Fluor oder
Chlor als Halogen, Benzoyl oder p-Nitrobenzoyl sind,
entsteht.

2. Verfahren nach Anspruch 1 zur Herstellung eines Rhodomycin-Derivates der Formel I,
worin
R¹ H, C₁-C₄-Alkyl oder eine Acylschutzgruppe,
R² COOCH₃, O-Si(C₁-C₄-Alkyl)₃ oder eine O-Acylschutzgruppe,
R³ C₁-C₄-Alkyl und
R⁴ und R⁵ zusammen eine Tetraisopropyldisiloxan-1,3-diyl-Schutzgruppe und
Acylschutzgruppe Acetyl, Mono-, Di-, oder Trihalogenacetyl mit Fluor oder
Chlor als Halogen, Benzoyl oder p-Nitrobenzoyl sind.

3. Verfahren nach Anspruch 1 zur Herstellung eines Rhodomycin-Derivates der Formel I,
worin
R¹ H, C₁-C₄-Alkyl oder eine Acylschutzgruppe,
R² OH, COOCH₃, O-Si(C₁-C₄-Alkyl)₃ oder eine O-Acylschutzgruppe,
R³ C₁-C₄-Alkyl und
R⁴ und R⁵ H sind.

## Claims

1. A process for the preparation of a rhodomycin derivative of the formula I 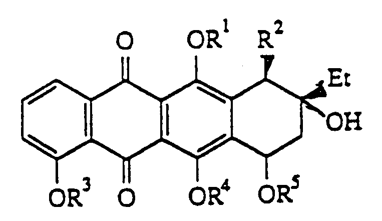 in which
R¹ is H, C₁-C₄-alkyl or an acyl protective group,
R² is OH, COOCH₃, O-Si(C₁-C₄-alkyl)₃ or an O-acyl protective group,
R³ is C₁-C₄-alkyl,
R⁴ and R⁵ are H or
R⁴ and R⁵ together are a tetraisopropyldisiloxane-1,3-diyl protective group and
acyl protective group is acetyl, mono-, di- or trihaloacetyl containing fluorine or chlorine as the halogen, benzoyl or p-nitrobenzoyl,
which comprises reacting a rhodomycinone derivative of the formula I,
in which
R¹ is H,
R² is OH or COOCH₃,
R³, R⁴ and R⁵ are H
with 1,3-dichloro-1,1,3,3-tetraisopropyldisiloxane in the presence of a base such as pyridine or dimethylaminopyridine and of an organic solvent such as dichloromethane or chloroform at a temperature between 15°C and 80°C to give a (6,7-tetraisopropyldisiloxane-1,3-diyl)rhodomycinone compound of the formula I and then reacting the compound which contains the radical R² = OH on the C-10 atom with tri-(C₁-C₄)alkylsilyl halide under the same reaction conditions, then alkylating the silylated intermediate compound of the formula I,
in which
R¹ and R³ are H,
R² is COOCH₃ or O-Si(C₁-C₄-alkyl)₃ and
R⁴ and R⁵ together are a tetraisopropyldisiloxane-1,3-diyl group,
with C₁-C₄-alkyl bromide or iodide in the presence of an alkali metal carbonate and of an organic solvent such as acetone or dioxane to give a mono- or dialkyl compound of the formula I,
in which
R¹ is C₁-C₄-alkyl,
R³ is H or C₁-C₄ alkyl and
R², R⁴ and R⁵ remain unchanged, then, in the case of a compound which contains an O-trialkylsilyl group on the C-10 atom, removing this group selectively by acid hydrolysis, preferably using hydrochloric acid, and acylating the resulting compound where the radical R² is a hydroxy group with an acylating agent selected from the group comprising acetyl chloride, mono-, di- or trihaloacetyl chloride or anhydride with fluorine or chlorine as the halogen, benzoyl chloride or p-nitrobenzoyl chloride, in the presence of a base such as pyridine, triethylamine or dimethylaminopyridine and of a solvent such as dichloromethane or chloroform to give a mono- or diacyl compound and then, in the case of a tetraisopropyldisiloxane compound of the formula I,
in which
R¹ is H, C₁-C₄-alkyl or an acyl protective group,
R² is COOCH₃ or an O-acyl protective group,
R³ is C₁-C₄-alkyl and
R⁴ and R⁵ together are a tetraisopropyldisiloxane-1,3-diyl protective group, removing the tetraisopropyldisiloxane-1,3-diyl protective group by means of an ammonium fluoride, preferably tetrabutylammonium fluoride, in a polar solvent such as tetrahydrofuran or dioxane, a compound of the formula I,
in which
R¹ is H, C₁-C₄-alkyl or an acyl protective group,
R² is COOCH₃ or an O-acyl protective group,
R³ is C₁-C₄-alkyl,
R⁴ and R⁵ are H and
acyl protective group is acetyl, mono-, di- or trihaloacetyl with fluorine or chlorine as the halogen, benzoyl or p-nitrobenzoyl,
resulting.

2. The process as claimed in claim 1 for the preparation of a rhodomycin derivative of the formula I,
in which
R¹ is H, C₁-C₄-alkyl or an acyl protective group,
R² is COOCH₃, O-Si(C₁-C₄-alkyl)₃ or an O-acyl protective group,
R³ is C₁-C₄-alkyl and
R⁴ and R⁵ together are a tetraisopropyldisiloxane-1,3-diyl protective group and
acyl protective group is acetyl, mono-, di- or trihaloacetyl with fluorine or chlorine as the halogen, benzoyl or p-nitrobenzoyl.

3. The process as claimed in claim 1 for the preparation of a rhodomycin derivative of the formula I, in which
R¹ is H, C₁-C₄-alkyl or an acyl protective group, R² is OH, COOCH₃, O-Si(C₁-C₄-alkyl)₃ or an O-acyl protective group,
R³ is C₁-C₄-alkyl and
R⁴ and R⁵ are H.

## Revendications

1. Procédé de préparation d'un dérivé de rhodomycine de formule I : 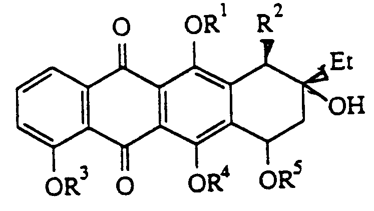 dans laquelle :
R¹ est H, un groupe alkyle en C₁-C₄ ou un groupe de protection acyle,
R² est OH, COOCH₃, O-Si(alkyle C₁-C₄)₃ ou un groupe de protection O-acyle,
R₃ est un groupe alkyle en C₁-C₄,
R⁴ ou R⁵ sont H ou
R⁴ et R⁵ conjointement sont un groupe de protection de tétraisopropyldisiloxane-1,3-diyle et
le groupe de protection acyle est un groupe acétyle, mono, di- ou trihalogénacétyle avec du fluor ou du chlore à titre d'halogène, benzoyle ou p-nitrobenzoyle,
caractérisé en ce que l'on fait réagir un dérivé de rhodomycinone de formule I, dans laquelle :
R¹ est H,
R² est OH ou COOCH₃,
R², R⁴ et R⁵ sont H,
avec du 1,3-dichloro-1,1,3,3-tétraisopropyldisiloxane en présence d'une base telle que la pyridine ou la diméthylaminopyridine et d'un solvant organique tel que le dichlorométhane ou le chloroforme à une température entre 15 et 80°C pour obtenir un composé 6,7-tétraisopropyldisiloxane-1,3-diyl)-rhodomycynone de formule I, ensuite le composé, qui contient le radical R² = OH sur l'atome C-10 est mis en réaction dans les mêmes conditions réactionnelles avec un halogénure de tri-(C₁-C₄)-alkylsilyle, puis on alkyle le composé intermédiaire silylé de formule I dans lequel :
R¹ et R³ sont H,
R² est COOCH₃ ou O-Si(alkyle C₁-C₄)₃ et
R⁴ et R⁵ sont conjointement un groupe de tétraisopropyldisiloxane-1,3-diyle,
avec du bromure ou de l'iodure d'alkyle en C₁-C₄ en présence d'un carbonate alcalin et d'un solvant organique tel que l'acétone ou le dioxanne en un composé mono ou dialkylé de formule I, dans lequel :
R¹ est un groupe alkyle en C₁-C₄,
R³ est H ou un groupe alkyle en C₁-C₄ et
R², R⁴ et R⁵ restent inchangés,
puis, dans un composé qui contient un groupe O-trialkylsilyle sur l'atome C-10, on sépare ce groupe de manière sélective par voie d'hydrolyse à l'acide, de préférence avec de l'acide chlorhydrique, et on acyle le composé obtenu dont le radical R² est un groupe hydroxy avec un agent d'acylation choisi parmi le chlorure d'acétyle, le chlorure ou l'anhydride de mono, di- ou trihalogénacétyle avec du fluor ou du chlore à titre d'halogène, le chlorure de benzoyle ou le chlorure de p-nitrobenzoyle en présence d'une base telle que la pyridine, la triéthylamine ou la diméthylaminopyridine et d'un solvant tel que le dichlorométhane ou le chloroforme pour former un composé mono ou diacylé et ensuite, dans un composé
tétraisopropyldisiloxane de formule I dans laquelle :
R¹ est H, un groupe alkyle en C₁-C₄ ou un groupe de protection acyle,
R² est COOCH₃ ou un groupe de protection O-acyle,
R³ est un groupe alkyle en C₁-C₄,
R⁴ et R⁵ conjointement sont un groupe de protection de tétraisopropyldisiloxane-1,3-diyle, on sépare le groupe de protection de tétraisopropyldisiloxane-1,3-diyle à l'aide d'un fluorure d'ammonium, de préférence du fluorure de tétrabutylammonium, dans un solvant polaire comme le tétrahydrofuranne ou le dioxanne, en sorte d'obtenir un composé de formule I dans lequel :
R¹ est H, un groupe alkyle en C₁-C₄ ou un groupe de protection acyle,
R² est COOCH₃ ou un groupe de protection O-acyle,
R³ est un groupe alkyle en C₁-C₄,
R⁴ et R⁵ sont H et
groupe de protection acyle est un groupe acétyle, mono, di- ou trihalogénacétyle avec du fluor ou du chlore à titre d'halogène, benzoyle ou p-nitrobenzoyle.

2. Procédé selon la revendication 1 pour la préparation d'un dérivé de rhodomycine de formule I dans laquelle :
R¹ est H, un groupe alkyle en C₁-C₄ ou un groupe de protection acyle,
R² est COOCH₃, O-Si(alkyle C₁-C₄)₃ ou un groupe de protection O-acyle,
R³ est un groupe alkyle en C₁-C₄ et
R⁴ et R⁵ sont conjointement un groupe de protection de tétraisopropyldisiloxane-1,3-diyle et
le groupe de protection acyle est un groupe acétyle, mono, di- ou trihalogénoacétyle avec un fluor ou du chlore à titre d'halogène, benzoyle ou p-nitrobenzoyle.

3. Procédé selon la revendication 1 pour la préparation d'un dérivé de rhodomycine de formule I dans laquelle :
R¹ est H, un groupe alkyle en C₁-C₄ ou un groupe de protection acyle,
R² est OH, COOCH₃, O-Si(alkyle C₁-C₄)₃ ou un groupe de protection O-acyle,
R³ est un groupe alkyle en C₁-C₄,
R⁴ et R⁵ sont H.
